# EUROPEAN PATENT SPECIFICATION

(11) **EP 3 668 549 B1**
(45) Date of publication and mention of the grant of the patent: **15.05.2024**
(21) Application number: 18773248.2
(22) Date of filing: 10.08.2018
(51) Int. Cl.: A61K 47/54, A61K 38/17, C07K 14/775, A61P 3/06, A61P 9/10

(54) **APOMERS**
APOMERE
APOMÈRES

(30) Priority: 10.08.2017 US 201762543466 P
(43) Date of publication of application: 24.06.2020
(73) Proprietor: Abionyx Pharma SA, 31130 Balma (FR)
(72) Inventor: DASSEUX, Jean-Louis, 31300 Toulouse (FR)
(74) Representative: Hoffmann Eitle
(86) International application number: PCT/IB2018/001060
(87) International publication number: WO 2019/030575

(56) References cited:
- WO-A1-2006/100567
- WO-A1-2009/158678
- WO-A1-2010/093918
- WO-A1-2012/109162
- WO-A1-2016/019333

## Description

### 1. CROSS-REFERENCE TO RELATED APPLICATIONS

This application claims the priority benefit of U.S. provisional application no. 62/543,466, filed August 10, 2017.

### 2. SEQUENCE LISTING

The instant application contains a Sequence Listing which has been submitted electronically in ASCII format. Said ASCII copy, created on August 3, 2018 is named CRN-019WO_ST25.txt and is 4,694 bytes in size.

### 3. BACKGROUND

Circulating cholesterol is carried by plasma lipoproteins - complex particles of lipid and protein - that transport lipids in the blood. Four major classes of lipoprotein particles circulate in plasma and are involved in the fat-transport system: chylomicrons, very low density lipoprotein (VLDL), low density lipoprotein (LDL) and high density lipoprotein (HDL). Chylomicrons constitute a short-lived product of intestinal fat absorption. VLDL and, particularly, LDL are responsible for the delivery of cholesterol from the liver (where it is synthesized or obtained from dietary sources) to extrahepatic tissues, including the arterial walls. HDL, by contrast, mediates reverse cholesterol transport (RCT), the removal of cholesterol lipids, in particular from extrahepatic tissues to the liver, where it is stored, catabolized, eliminated or recycled. HDL also plays a beneficial role in inflammation, transporting oxidized lipids and interleukin, which may in turn reduce inflammation in blood vessel walls.

Cardiovascular diseases such as coronary heart disease, coronary artery disease and atherosclerosis are linked overwhelmingly to elevated serum cholesterol levels. For example, atherosclerosis is a slowly progressive disease characterized by the accumulation of cholesterol within the arterial wall. Compelling evidence supports the theory that lipids deposited in atherosclerotic lesions are derived primarily from plasma LDLs; thus, LDLs have popularly become known as "bad" cholesterol. In contrast, High density lipoprotein (HDL) serum levels correlate inversely with coronary heart disease. Indeed, high serum levels of HDLs are regarded as a negative risk factor. As a consequence, HDLs have popularly become known as "good" cholesterol, (see, *e*.*g*., Zhang, et al., 2003 Circulation 108:661-663). Many companies have developed HDL mimetics based on human Apolipoprotein A-I ("ApoA-I")/phospholipid complexes or based on apolipoprotein peptide analogues/phospholipid complexes (*e*.*g*., CER-001 developed by Cerenis Therapeutics (see, *e.g.,* Tardy et al., 2014, Atherosclerosis 232(1):110-118); MDCO-216, formerly known as ETC-216, developed by The Medicines Company (see, *e*.*g*., Kallend et al., Eur Heart J Cardiovasc Pharmacother. (1):23-9); CSL111 and CSL1 12 developed by CSL Behring (see, *e.g.,* Diditchenko et al., 2016, Circ Res 119(6):751-763)). However, demonstration of clinical efficacy in regression of atherosclerotic plaques has been challenging and clinical results were not always positive (see, *e*.*g*., Tardif et al., 2007, JAMA 297(15):1675-82), Tardif et al., 2014, Eur. Heart J. 35:3277-3286).

Recent studies have challenged HDL-cholesterol as a predictive factor for cardiovascular disease and support the number of HDL particles as a better predictor of the risk of cardiovascular disease (see, Jensen and Miedema, 2017, www.acc.org/latest-in-cardiology/articles/2017/02/01/07/34/quality-over-quantity; Qi et al, 2015, J Am Coll Cardiol. 65(4):355-363; Mackey et al., 2012, J Am Coll Cardiol. 60(6):508-16; deGoma and Rader, J Am Coll Cardiol 60(6):517-520). It has been demonstrated that levels of ApoA-I, the major protein component of HDL, is negatively correlated with the risk of cardiovascular disease, and that ApoA-I levels are in fact a better predictor of the risk of cardiovascular disease than HDL-cholesterol (Luc et al., 2002, Arteriosclerosis, Thrombosis, and Vascular Biology 22:1155-1161). Circulating levels of ApoA-I are associated with cholesterol efflux capacity (Borja et al., 2015, The Journal of Lipid Research, 56:2002-2009). The ATP-binding cassette transporter A1 (ABCA1) is responsible for cellular cholesterol efflux to lipid-free ApoA-I and lipid-poor ApoA-I, rather than to discoidal HDL, and they are thought to be the primary acceptors of cellular cholesterol and phospholipids via ABCA1 (Jayaraman et al., 2012, Biochem J. 442(3): 703-12). ABCA1 mediated cholesterol efflux capacity mediated by ApoA-I and lipid-poor ApoA-I is predictive of the risk of cardiovascular disease (Mody et al., 2016, J Am Coll Cardiol. 67(21):2480-7; Khera et al., 2011, New England Journal of Medicine 364:127-135).

It has been speculated that HDL mimetics such as CSL-112 or MDCO-216 could be protective against cardiovascular disease because of their ability to fuse with endogenous HDL, liberating lipid-poor ApoA-I after perfusion in patients (Diditchenko et al., 2016, Circ Res 119(6):751-763; Diditchenko et al., 2013 Arterioscler Thromb Vasc Biol 3(9):2202-11). However, such a mechanism of action is subject to question as MDCO-216 failed to show plaque regression in clinical trials (www.themedicinescompany.com/investors/news/medicines-company-discontinues-development-mdco-216-its-investigational-cholesterol). One might speculate that there is a need to have a drug that will boost ABCA1 mediated cholesterol efflux capacity without being associated with the drawbacks of HDL, namely the presence of phospholipids, which can potentially be pro-atherogenic. The ideal would be to be able to administer an ApoA-I drug alone, which is quite difficult as ApoA-I has a tendency to aggregate in water based buffer solutions. The only clinical trial that tested administration of lipid-free ApoA-I resulted in increased triglycerides and VLDL, which later was interpreted as a result of ApoA-I aggregates (Nanjee et al., 1996, Arteriosclerosis, Thrombosis, and Vascular Biology 16:1203-1214). Apomer complexes with apolipoprotein and an high amount of amphipathic molecules such as lipids have also been disclosed in the art (cf. WO 2012/109162 A1). These have been proposed for the treatment of dyslipidemic disorders.

Thus, there is a need for new compositions of lipid-poor apolipoproteins that are suitable for treating dyslipidemic disorders or hepatic disorders. Such compositions should ideally avoid any apolipoprotein aggregation (which can lead to non-functional complexes), promote cellular cholesterol efflux, and have low liver toxicity.

### 4. SUMMARY

This disclosure relates to lipid-poor complexes comprising an apolipoprotein in monomeric or multimeric form complexed with amphipathic molecules, referred to herein as "Apomers".

Accordingly, the present invention relates to a composition comprising a population of Apomers for use as a medicament, each Apomer comprising 1-8 apolipoprotein molecules complexed with amphipathic molecules, wherein:
(i) the amphipathic molecules contribute a net charge of at least +1 or -1 per molecule of apolipoprotein; and
(ii) the molar ratio of apolipoprotein molecules to amphipathic molecules ranges from 8:1 to 1:15.

Other embodiments of the present invention are as defined in the appended claims.

Apomers are believed to provide several advantages over discoidal HDL mimetic lipoprotein complexes. Administration of an Apomer can be a more efficient way to promote cholesterol efflux compared to HDL mimetics because Apomers, in contrast to HDL mimetic lipoprotein complexes, are lipid-poor, thereby avoiding the need to form lipid-poor apolipoproteins *in vivo.* Apomers may also be safer than HDL mimetic lipoprotein complexes, as the lipid load of Apomers is minimal, thereby avoiding potential pro-atherogenic effects of lipid overload that may result from administration of HDL mimetic lipoprotein complexes. Moreover, the amount of apolipoprotein administered via Apomers can be increased relative to the amount of apolipoprotein that can be administered via HDL mimetic lipoprotein complexes because Apomers contain a higher percentage of apolipoprotein as compared to HDL mimetic lipoprotein complexes. Apomers are also believed to have different pharmacokinetics and pharmacodynamics (for instance an increased ApoA-I circulation time) compared to HDL mimetic lipoprotein complexes, and can penetrate the blood brain barrier and be taken up into the lymph, providing additional advantages over discoidal particles. Apomers can provide additional advantages over lipid-free ApoA-I, as they can have less of a tendency to form non-functional aggregates which are unable to dissociate *in vivo.* Moreover, Apomers can be administered to a subject by multiple routes, for example, by intravenous, sub-cutaneous, intraperitoneal, and intra-muscular injection. Moreover, Apomers can alleviate the patient burden associated with intravenous administration when administered by a sub-cutaneous route or an intra-muscular route because such administrations can be performed by a patient without a nurse or physician. Depot forms can also be used to avoid the need for multiple administrations or to provide less frequent dosing. Finally, the lower amount of phospholipids in Apomers compared to discoidal HDL particles results in lower production costs compared to discoidal HDL.

Generally, Apomers comprise one or more apolipoprotein molecules, each complexed with one or more amphipathic molecules. In certain aspects, the amphipathic molecules together contribute a net charge of at least +1 or -1 per apolipoprotein molecule in an Apomer. Exemplary apolipoproteins that can be used in the Apomers of the disclosure are described in Section 6.1.1. Exemplary amphipathic molecules are described in Section 6.1.2.

The disclosure further provides compositions comprising an Apomer of the disclosure, including pharmaceutical compositions. Exemplary compositions are described in Section 6.2.

The disclosure further provides the Apomers for use in methods of treating a subject that comprise administering a therapeutically effective amount of an Apomer or a pharmaceutical composition of the disclosure to the subject. Exemplary methods of treating a subject are described in Section 6.3.

### 5. BRIEF DESCRIPTION OF THE FIGURES

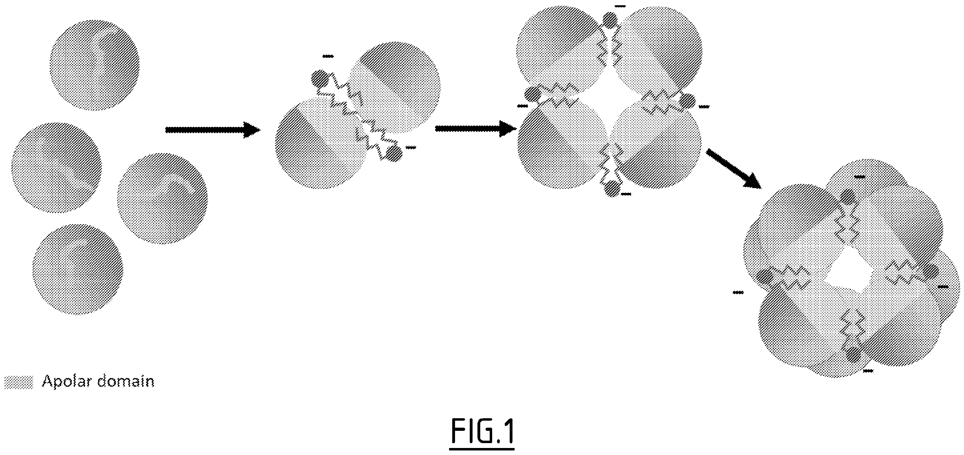

**FIG. 1** is a schematic showing a process by which the inventor believes Apomers comprising 8 apolipoprotein molecules and negatively charged amphipathic molecules are formed. Without being bound by theory, it is believed that Apomers having 1 apolipoprotein molecule are formed first from apolipoprotein monomers and amphipathic molecules. Apomers with 2 apolipoprotein molecules (*i*.*e*., Apomers with apolipoprotein dimers) are then formed by dimerization of Apomers having 1 apolipoprotein molecule, and Apomers having 4 apolipoprotein molecules (*i*.*e*., Apomers with apolipoprotein tetramers) are then formed by dimerization of Apomers with 2 apolipoprotein molecules. Finally, Apomers with 8 lipoprotein molecules (*i*.*e*., Apomers with apolipoprotein octamers) are formed by dimerization of the Apomers having 4 apolipoprotein molecules. Although the arrows shown in Fig. 1 are depicted in one direction, the formation of Apomers are believed to result in the formation of different species that are present in an equilibrium. The equilibrium can be influenced by conditions such as pH, concentration, ionic strength, and temperature, *e*.*g*., as described in Section 6.1. It should be understood that the numbers of the component molecules shown in Fig. 1 are merely illustrative and that Apomers having different ratios of component molecules are contemplated.
**FIG. 2** shows an overlay of gel permeation chromatograms of human ApoA-I, CER-001, and Apomers comprising human ApoA-I, 1,2-dipalmitoyl-*sn*-glycero-3-[phospho-*rac*-(1-glycerol)] (DPPG) and sphingomyelin in a 1:2:2 molar ratio.
**FIG. 3** shows plasma levels of human ApoA-I in rabbits following intravenous and sub-cutaneous administration of Apomers comprising human ApoA-I, 1,2-dipalmitoyl-*sn-*glycero-3-[phospho-*rac*-(1-glycerol)] (DPPG) and sphingomyelin in a 1:2:2 molar ratio.

### 6. DETAILED DESCRIPTION

### 6.1. Apomers

The Apomers of the disclosure comprise 1-8 apolipoprotein molecules (*e*.*g*., 1, 2, 4, or 8 lipoprotein molecules) complexed with a sufficient number of amphipathic molecules to solubilize the apolipoprotein molecules.

The apolipoprotein molecules are complexed with the amphipathic molecules in an apolipoprotein:amphipathic molecule molar ratio ranging from 8:1 to 1:15 (*e*.*g*., from 8:1 to 1:15, from 7:1 to 1:15, from 6:1 to 1:15, from 5:1 to 1:15, from 4:1 to 1:15, from 3:1 to 1:15, from 2:1 to 1:15, from 1:1 to 1:15, from 8:1 to 1:14, from 7:1 to 1:14, from 6:1 to 1:14, from 5:1 to 1:14, from 4:1 to 1:14, from 3:1 to 1:14, from 2:1 to 1:14, from 1:1 to 1:14, from 8:1 to 1:13, from 7:1 to 1:13, from 6:1 to 1:13, from 5:1 to 1:13, from 4:1 to 1:13, from 3:1 to 1:13, from 2:1 to 1:13, from 1:1 to 1:13, from 8:1 to 1:12, from 7:1 to 1:12, from 6:1 to 1:12, from 5:1 to 1:12, from 4:1 to 1:12, from 3:1 to 1:12, from 2:1 to 1:12, from 1:1 to 1:12, from 8:1 to 1:11, from 7:1 to 1:11, from 6:1 to 1:11, from 5:1 to 1:11, from 4:1 to 1:11, from 3:1 to 1:11, from 2:1 to 1:11, from 1:1 to 1:11, from 8:1 to 1:10, from 7:1 to 1:10, from 6:1 to 1:10, from 5:1 to 1:10, from 4:1 to 1:10, from 3:1 to 1:10, from 2:1 to 1:10, from 1:1 to 1:10, from 8:1 to 1:9, from 7:1 to 1:9, from 6:1 to 1:9, from 5:1 to 1:9, from 4:1 to 1:9, from 3:1 to 1:9, from 2:1 to 1:9, from 1:1 to 1:9, from 8:1 to 1:8, from 7:1 to 1:8, from 6:1 to 1:8, from 5:1 to 1:8, from 4:1 to 1:8, from 3:1 to 1:8, from 2:1 to 1:8, from 1:1 to 1:8, from 8:1 to 1:7, from 7:1 to 1:7, from 6:1 to 1:7, from 5:1 to 1:7, from 4:1 to 1:7, from 3:1 to 1:7, from 2:1 to 1:7, from 1:1 to 1:7, from 8:1 to 1:6, from 7:1 to 1:6, from 6:1 to 1:6, from 5:1 to 1:6, from 4:1 to 1:6, from 3:1 to 1:6, from 2:1 to 1:6, from 1:1 to 1:6, from 8:1 to 1:5, from 7:1 to 1:5, from 6:1 to 1:5, from 5:1 to 1:5, from 4:1 to 1:5, from 3:1 to 1:5, from 2:1 to 1:5, from 1:1 to 1:5, from 8:1 to 1:4, from 7:1 to 1:4, from 6:1 to 1:4, from 5:1 to 1:4, from 4:1 to 1:4, from 3:1 to 1:4, from 2:1 to 1:4, from 1:1 to 1:4, from 8:1 to 1:3, from 7:1 to 1:3, from 6:1 to 1:3, from 5:1 to 1:3, from 4:1 to 1:3, from 3:1 to 1:3, from 2:1 to 1:3, from 1:1 to 1:3, from 8:1 to 1:2, from 7:1 to 1:2, from 6:1 to 1:2, from 5:1 to 1:2, from 4:1 to 1:2, from 3:1 to 1:2, from 2:1 to 1:2, from 1:1 to 1:2, from 8:1 to 1:1, from 7:1 to 1:1, from 6:1 to 1:1, from 5:1 to 1:1, from 4:1 to 1:1, from 3:1 to 1:1, or from 2:1 to 1:1).

In some embodiments, the apolipoprotein molecules are complexed with the amphipathic molecules in a molar ratio ranging from 6:1 to 1:6 (*e*.*g*., from 5:1 to 1:6, from 4:1 to 1:6, from 3:1 to 1:6, from 2:1 to 1:6, from 5:1 to 1:5, from 4:1 to 1:5, from 3:1 to 1:5, from 2:1 to 1:5, from 5:1 to 1:4, from 4:1 to 1:4, from 3:1 to 1:4, from 2:1 to 1:4, from 5:1 to 1:3, from 4:1 to 1:3, from 3:1 to 1:3, from 2:1 to 1:3, from 5:1 to 1:2, from 4:1 to 1:2, from 3:1 to 1:2, from 2:1 to 1:2, from 5:1 to 1:1, from 4:1 to 1:1, from 3:1 to 1:1, from 2:1 to 1:1, from 1:1 to 1:6, from 1:1 to 1:5, from 1:1 to 1:4, from 1:1 to 1:3, from 1:1 to 1:2, from 1:2 to 1:6, from 1:2 to 1:5, from 1:2 to 1:4, from 1:2 to 1:3, from 1:3 to 1:6, from 1:3 to 1:5, from 1:3 to 1:4, from 1:4 to 1:6, from 1:4 to 1:5, from 1:5 to 1:6, from 1.5:1 to 1:2, from 5:4 to 4:5, from 5:3 to 3:5, from 5:2 to 2:5, or from 3:2 to 2:3).

The molar ratio of apolipoprotein molecules to amphipathic molecules can be, but does not necessarily have to be in integers or reflect a one to one relationship between the apolipoprotein and amphipathic molecules. By way of example and not limitation, an Apomer can have an apolipoprotein to amphipathic molecule molar ratio of 2:5, 8:7, 3:2, or 4:7. The amphipathic molecules can together contribute a net charge of at least +1 or - 1 per apolipoprotein in the Apomer (*e*.*g*., +1, +2, +3, -1, -2, or -3). In some embodiments, the net charge is a negative charge. In other embodiments, the net charge is a positive charge. Unless required otherwise by context, charge is measured at physiological pH.

Apomers of the disclosure can be made, for example, by combining and mixing a composition comprising apolipoprotein molecules (*e*.*g*., a composition comprising multimer aggregates of apolipoprotein) with a solution comprising the amphipathic molecules until a solution of Apomers is formed.

For example, Apomers can be prepared by mixing two organic solutions, one containing an apolipoprotein and the other one containing a charged amphipathic molecule, then removing the solvent by methods such as evaporation, freeze-drying (lyophilization), heating or any other methods known in the art. Apomers can also be prepared by mixing two aqueous solutions, one containing an apolipoprotein and the other one containing a charged amphipathic molecule, until an homogeneous solution is obtained. Apomers can also be prepared by hydrating an apolipoprotein by an aqueous solution of charged amphipathic molecules, then mixing until an homogeneous solution is obtained. The solutions used to make Apomers, *e*.*g*., aqueous solutions, can be at room temperature, at a higher temperature than room temperature, or at a lower temperature than room temperature during formation of the Apomers. Alternatively, the solutions can be thermal cycled between a higher and lower temperature, *e*.*g*., as described in Example 1 of WO 2012/109162, preferably until Apomers of at least 85%, at least 90%, at least 95% or at least 98% homogeneity are obtained.

Without being bound by theory, it is believed that the process of making Apomers results in the formation of multiple species of Apomers having different numbers of apolipoprotein molecules in equilibrium. It is known in the art that the self-association of lipid-free ApoA-I is influenced by conditions such as pH, concentration, ionic strength, and temperature (see, *e.g.,* Gianazza et al., 1997, Biochemistry, 36:7898-7905; Jayaraman et al., Journal of Biological Chemistry, 286(41):35610-35623; Schonfeld et al., 2016 J. Phys. Chem. B, 120:1228-1235) and it is believed that the equilibrium between different Apomer species is similarly influenced by pH, concentration, ionic strength, and temperature. For example, acidic pH promotes formation of monomeric ApoA-I whereas alkaline pH encourages formation of multimeric forms, low concentrations of ApoA-I favor monomeric ApoA-I whereas high concentrations favor multimeric forms, and monomeric forms of ApoA-I are favored as temperature increases or decreases from ApoA-I's self-association maximum of 22°C.

In some embodiments, the ratio of the apolipoprotein molecules to amphipathic molecules is about 1:1. In other embodiments, the ratio of the apolipoprotein molecules to amphipathic molecules is about 1:2. In yet other embodiments, the ratio of the apolipoprotein molecules to amphipathic molecules is about 1:3. In yet other embodiments, the ratio of the apolipoprotein molecules to amphipathic molecules is about 1:4. In yet other embodiments, the ratio of the apolipoprotein molecules to amphipathic molecules is about 1:5. In yet other embodiments, the ratio of the apolipoprotein molecules to amphipathic molecules is about 1:6.

In some embodiments, an Apomer comprises 1 apolipoprotein molecule.

In other embodiments, an Apomer comprises 2 apolipoprotein molecules. Apomers comprising 2 apolipoprotein molecules preferably have a Stokes radius of 3 nm or less. In some embodiments, an Apomer can comprise 2 apolipoprotein molecules and 1, 2, or 3 negatively charged amphipathic molecules (*e*.*g*., negatively charged phospholipid molecules) per apolipoprotein molecule.

In other embodiments, an Apomer comprises 4 apolipoprotein molecules. Apomers comprising 4 apolipoprotein molecules preferably have a Stokes radius of 4 nm or less. In some embodiments, an Apomer can comprise 4 apolipoprotein molecules and 1, 2, or 3 negatively charged amphipathic molecules (*e*.*g*., negatively charged phospholipid molecules) per apolipoprotein molecule.

In other embodiments, an Apomer comprises 8 apolipoprotein molecules. Apomers comprising 8 apolipoprotein molecules preferably have a Stokes radius of 5 nm or less. In some embodiments, an Apomer can comprise 8 apolipoprotein molecules and 1, 2, or 3 negatively charged amphipathic molecules (*e*.*g*., negatively charged phospholipid molecules) per apolipoprotein molecule.

The Apomers of the disclosure can contain one or more sterols and/or sterol derivatives (*e*.*g*., a plant sterol, an animal sterol, or a sterol derivative such as a vitamin). For example, an Apomer can contain cholesterol or a cholesterol derivative, *e*.*g*., a cholesterol ester. The cholesterol derivative can also be a substituted cholesterol or a substituted cholesterol ester. The Apomers of the disclosure can also contain an oxidized sterol such as, but not limited to, oxidized cholesterol or an oxidized sterol derivative (such as, but not limited to, an oxidized cholesterol ester).

In certain embodiments, the Apomers of the disclosure do not contain cholesterol and/or a cholesterol derivative (*e*.*g*., as described in the preceding paragraph, for example a cholesterol ester).

The Apomers of the disclosure are preferably soluble in a biological fluid, for example one or more of lymph, cerebrospinal fluid, vitreous humor, aqueous humor, and blood or a blood fraction (*e*.*g*., serum or plasma).

Apomers may include a targeting functionality, for example to target the Apomers to a particular cell or tissue type. In some embodiments, an Apomer includes a targeting moiety attached to an apolipoprotein molecule or an amphipathic molecule.

### 6.1.1. Apolipoproteins

Suitable apolipoproteins that can be included in the Apomers of the disclosure include apolipoproteins ApoA-I, ApoA-II, ApoA-IV, ApoA-V, ApoB, ApoC-I, ApoC-II, ApoC-III, ApoD, ApoE, ApoJ, ApoH, and any combination of two or more of the foregoing. Polymorphic forms, isoforms, variants and mutants as well as truncated forms of the foregoing apolipoproteins, the most common of which are Apolipoprotein A-I_{Milano} (ApoA-I_{M}), Apolipoprotein A-Iₚₐᵣᵢₛ (ApoA-I_{P}), and Apolipoprotein A-I_{Zaragoza} (ApoA-I_{Z}), can also be used. Apolipoproteins mutants containing cysteine residues are also known, and can also be used (see, *e*.*g*., U.S. Publication No. 2003/0181372). The apolipoproteins can be modified in their primary sequence to render them less susceptible to oxidations, for example, as described in U.S. Publication Nos. 2008/0234192 and 2013/0137628, and U.S. Patent Nos. 8,143,224 and 8,541,236. The apolipoproteins can include residues corresponding to elements that facilitate their isolation, such as His tags, or other elements designed for other purposes. Preferably, the apolipoprotein in the Apomer is soluble in a biological fluid (*e*.*g*., lymph, cerebrospinal fluid, vitreous humor, aqueous humor, blood or a blood fraction (*e*.*g*., serum or plasma)).

Apolipoproteins can be purified from animal sources (and in particular from human sources) or produced recombinantly as is well-known in the art, see, *e.g.,* Chung et al., 1980, J. Lipid Res. 21 (3):284-91; Cheung et al., 1987, J. Lipid Res. 28(8):913-29. See also U.S. Patent Nos. 5,059,528, 5,128,318, 6,617,134; U.S. Publication Nos. 2002/0156007, 2004/0067873, 2004/0077541, and 2004/0266660; and PCT Publications Nos. WO 2008/104890 and WO 2007/023476. Other methods of purification are also possible, for example as described in PCT Publication No. WO 2012/109162.

The apolipoprotein can be in prepro- form, pro- form, or mature form. For example, an Apomer can comprise ApoA-I (*e*.*g*., human ApoA-I) in which the ApoA-I is preproApoA-I, proApoA-I, or mature ApoA-I. In some embodiments, an Apomer comprises ApoA-I that has at least 90% sequence identity to SEQ ID NO:2. In other embodiments, an Apomer comprises ApoA-I that has at least 95% sequence identity to SEQ ID NO:2. In other embodiments, an Apomer comprises ApoA-I that has at least 98% sequence identity to SEQ ID NO:2. In other embodiments, an Apomer comprises ApoA-I that has at least 99% sequence identity to SEQ ID NO:2. In other embodiments, an Apomer comprises ApoA-I that has 100% sequence identity to SEQ ID NO:2.

The apolipoprotein molecule(s) can comprise a chimeric apolipoprotein comprising an apolipoprotein and one or more attached functional moieties, such as for example, one or more targeting moieties, a moiety having a desired biological activity, an affinity tag to assist with purification, and/or a reporter molecule for characterization or localization studies. In one embodiment, an attached functional moiety of a chimeric apolipoprotein is not in contact with hydrophobic surfaces of the Apomer. In another embodiment, an attached functional moiety is in contact with hydrophobic surfaces of the Apomer. In some embodiments, a functional moiety of a chimeric apolipoprotein may be intrinsic to a natural protein. In some embodiments, a chimeric apolipoprotein includes a ligand or sequence recognized by or capable of interaction with a cell surface receptor or other cell surface moiety.

In one embodiment, a chimeric apolipoprotein includes a targeting moiety that is not intrinsic to the native apolipoprotein, such as for example, *S. cerevisiae* α-mating factor peptide, folic acid, transferrin, or lactoferrin. In one embodiment, a chimeric apolipoprotein may include a functional moiety intrinsic to an apolipoprotein. One example of an apolipoprotein intrinsic functional moiety is the intrinsic targeting moiety formed approximately by amino acids 130-150 of human ApoE, which comprises the receptor binding region recognized by members of the low density lipoprotein receptor family. Other examples of apolipoprotein intrinsic functional moieties include the region of ApoB-100 that interacts with the low density lipoprotein receptor and the region of ApoA-I that interacts with scavenger receptor type B 1. In other embodiments, a functional moiety may be added synthetically or recombinantly to produce a chimeric apolipoprotein. Another example is an apolipoprotein with the prepro or pro sequence from another preproapolipoprotein (*e*.*g*., prepro sequence from preproapoA-II substituted for the prepro sequence of preproapoA-I). Another example is an apolipoprotein for which some of the amphipathic sequence segments have been substituted by other amphipathic sequence segments from another apolipoprotein.

As used herein, "chimeric" refers to two or more molecules that are capable of existing separately and are joined together to form a single molecule having the desired functionality of all of its constituent molecules. The constituent molecules of a chimeric molecule may be joined synthetically by chemical conjugation or, where the constituent molecules are all polypeptides or analogs thereof, polynucleotides encoding the polypeptides may be fused together recombinantly such that a single continuous polypeptide is expressed. Such a chimeric molecule is termed a fusion protein. A "fusion protein" is a chimeric molecule in which the constituent molecules are all polypeptides and are attached (fused) to each other such that the chimeric molecule forms a continuous single chain. The various constituents can be directly attached to each other or can be coupled through one or more linkers. One or more segments of various constituents can be, for example, inserted in the sequence of an apolipoprotein, or, as another example, can be added N-terminal or C-terminal to the sequence of an apolipoprotein.

In some embodiments, a chimeric apolipoprotein is prepared by chemically conjugating the apolipoprotein and the functional moiety to be attached. Means of chemically conjugating molecules are well known to those of skill in the art. Such means will vary according to the structure of the moiety to be attached, but will be readily ascertainable to those of skill in the art. Polypeptides typically contain a variety of functional groups, *e*.*g*., carboxylic acid (--COOH), free amino (--NH2), or sulfhydryl (--SH) groups, that are available for reaction with a suitable functional group on the functional moiety or on a linker to bind the moiety thereto. A functional moiety may be attached at the N-terminus, the C-terminus, or to a functional group on an interior residue (*i.e.,* a residue at a position intermediate between the N- and C-termini) of an apolipoprotein molecule. Alternatively, the apolipoprotein and/or the moiety to be tagged can be derivatized to expose or attach additional reactive functional groups.

In some embodiments, fusion proteins that include a polypeptide functional moiety are synthesized using recombinant expression systems. Typically, this involves creating a nucleic acid (*e*.*g*., DNA) sequence that encodes the apolipoprotein and the functional moiety such that the two polypeptides will be in frame when expressed, placing the DNA under the control of a promoter, expressing the protein in a host cell, and isolating the expressed protein.

A nucleic acid encoding a chimeric apolipoprotein can be incorporated into a recombinant expression vector in a form suitable for expression in a host cell. As used herein, an "expression vector" is a nucleic acid which, when introduced into an appropriate host cell, can be transcribed and translated into a polypeptide. The vector may also include regulatory sequences such as promoters, enhancers, or other expression control elements *(e.g.,* polyadenylation signals). Such regulatory sequences are known to those skilled in the art (see, e.g., Goeddel, 1990, Gene Expression Technology: Meth. Enzymol. 185, Academic Press, San Diego, Calif.; Berger and Kimmel, Guide to Molecular Cloning Techniques, Methods in Enzymology 152 Academic Press, Inc., San Diego, Calif.; Sambrook et al., 1989, Molecular Cloning--A Laboratory Manual (2nd ed.) Vol. 1-3, Cold Spring Harbor Laboratory, Cold Spring Harbor Press, NY, etc.).

In some embodiments, an apolipoprotein has been modified such that when the apolipoprotein is incorporated into an Apomer, the modification will increase stability of the Apomer or confer targeting ability. In one embodiment, the modification includes introduction of cysteine residues into apolipoprotein molecules to permit formation of intramolecular or intermolecular disulfide bonds, *e.g.,* by site-directed mutagenesis. In another embodiment, a chemical crosslinking agent is used to form intermolecular links between apolipoprotein molecules to enhance stability of the Apomers. Intermolecular crosslinking prevents or reduces dissociation of apolipoprotein molecules from the Apomers and/or prevents displacement by endogenous apolipoprotein molecules within an individual to whom the Apomers are administered. In other embodiments, an apolipoprotein is modified either by chemical derivatization of one or more amino acid residues or by site directed mutagenesis, to confer targeting ability to or recognition by a cell surface receptor.

Apomers can be targeted to a specific cell surface receptor by engineering receptor recognition properties into an apolipoprotein. For example, Apomers may be targeted to macrophages by altering the apolipoprotein to confer recognition by the macrophage endocytic class A scavenger receptor (SR-A). SR-A binding ability can be conferred to an Apomer by modifying the apolipoprotein by site directed mutagenesis to replace one or more positively charged amino acids with a neutral or negatively charged amino acid. SR-A recognition can also be conferred by preparing a chimeric apolipoprotein that includes an N- or C-terminal extension having a ligand recognized by SR-A or an amino acid sequence with a high concentration of negatively charged residues. Apomers can also interact with apolipoprotein receptors such as, but not limited to, ABCA1 receptors, ABCG1 receptors, Megalin, Cubulin and HDL receptors such as SR-Bl.

### 6.1.2. Amphipathic molecules

An amphipathic molecule is a molecule that possesses both hydrophobic (apolar) and hydrophilic (polar) elements. Amphipathic molecules that can be used in the Apomers of the disclosure include lipids, detergents, fatty acids, and apolar molecules covalently attached to polar molecules such as, but not limited to, sugars or nucleic acids. The Apomers of the disclosure can include a single class of amphipathic molecule (*e*.*g*., a single species of phospholipids or a mixture of phospholipids), or can contain a combination of classes of amphipathic molecules (*e*.*g*., phospholipids and detergents). The Apomer can contain one species of amphipathic molecules or a combination of amphipathic molecules configured to facilitate solubilization of the apolipoprotein molecule(s).

### 6.1.2.1. Lipids

The Apomers of the disclosure can include one or more lipids. In various embodiments, one or more lipids can be saturated and/or unsaturated, natural and/or synthetic, charged or not charged, zwitterionic or not. Phospholipids can have two acyl chains that are the same or different (for example, chains having a different number of carbon atoms, a different degree of saturation between the acyl chains, different branching of the acyl chains, or a combination thereof). The lipid can also be modified to contain a fluorescent probe (*e*.*g*., as described at avantilipids.com/product-category/products/fluorescent-lipids/). Preferably, the lipid comprises at least one phospholipid.

Phospholipids can have unsaturated or saturated acyl chains ranging from about 6 to about 24 carbon atoms (*e*.*g*., 6-20, 6-16, 6-12, 12-24, 12-20, 12-16, 16-24, 16-20, or 20-24). In some embodiments, a phospholipid used in a Apomer has one or two acyl chains of 12, 14, 16, 18, 20, 22, or 24 carbons (*e*.*g*., two acyl chains of the same length).

Non-limiting examples of acyl chains present in commonly occurring fatty acids that can be included in phospholipids are provided in Table 1, below:

| **Table 1** | |
|---|---|
| Length:Number of Unsaturations | Common Name |
| 14:0 | myristic acid |
| 16:0 | palmitic acid |
| 18:0 | stearic acid |
| 18:1 cisΔ⁹ | oleic acid |
| 18:2 cisΔ^{9,12} | linoleic acid |
| 18:3 cisΔ^{9,12,15} | linonenic acid |
| 20:4 cisΔ^{5,8,11,14} | arachidonic acid |
| 20:5 cisaΔ^{5,8,11,14,17} | eicosapentaenoic acid (an omega-3 fatty acid) |

Lipids that can be present in the Apomers include, but are not limited to, small alkyl chain phospholipids, egg phosphatidylcholine, soybean phosphatidylcholine, dipalmitoylphosphatidylcholine, dimyristoylphosphatidylcholine, distearoylphosphatidylcholine 1-myristoyl-2-palmitoylphosphatidylcholine, 1-palmitoyl-2-myristoylphosphatidylcholine, 1-palmitoyl-2-stearoylphosphatidylcholine, 1-stearoyl-2-palmitoylphosphatidylcholine, dioleoylphosphatidylcholine dioleophosphatidylethanolamine, dilauroylphosphatidylglycerol phosphatidylcholine, phosphatidylserine, phosphatidylethanolamine, phosphatidylinositol, phosphatidylglycerols, diphosphatidylglycerols such as dimyristoylphosphatidylglycerol, dipalmitoylphosphatidylglycerol, distearoylphosphatidylglycerol, dioleoylphosphatidylglycerol, dimyristoylphosphatidic acid, dipalmitoylphosphatidic acid, dimyristoylphosphatidylethanolamine, dipalmitoylphosphatidylethanolamine, dimyristoylphosphatidylserine, dipalmitoylphosphatidylserine, brain phosphatidylserine, brain sphingomyelin, palmitoylsphingomyelin, dipalmitoylsphingomyelin, egg sphingomyelin, milk sphingomyelin, phytosphingomyelin, distearoylsphingomyelin, dipalmitoylphosphatidylglycerol salt, phosphatidic acid, galactocerebroside, gangliosides, cerebrosides, dilaurylphosphatidylcholine, (1,3)-D-mannosyl-(1,3)diglyceride, aminophenylglycoside, 3-cholesteryl-6'-(glycosylthio)hexyl ether glycolipids, and cholesterol and its derivatives. Synthetic lipids, such as synthetic palmitoylsphingomyelin or N-palmitoyl-4-hydroxysphinganine-1-phosphocholine (a form of phytosphingomyelin) can be used to minimize lipid oxidation.

In some embodiments, an Apomer includes two types of phospholipids: a neutral lipid, *e*.*g*., lecithin and/or sphingomyelin (abbreviated SM or SPH), and a charged phospholipid (*e*.*g*., a negatively charged phospholipid). A "neutral" phospholipid has a net charge of about zero at physiological pH. In many embodiments, neutral phospholipids are zwitterions, although other types of net neutral phospholipids are known and can be used. In some embodiments, the molar ratio of the charged phospholipid (*e*.*g*., negatively charged phospholipid) to neutral phospholipid ranges from 1:1 to 1:3, for example, about 1:1, about 1:2, or about 1:3.

The neutral phospholipid can comprise, for example, one or both of the lecithin and/or SM, and can optionally include other neutral phospholipids. In some embodiments, the neutral phospholipid comprises lecithin, but not SM. In other embodiments, the neutral phospholipid comprises SM, but not lecithin. In still other embodiments, the neutral phospholipid comprises both lecithin and SM. All of these specific exemplary embodiments can include neutral phospholipids in addition to the lecithin and/or SM, but in many embodiments do not include such additional neutral phospholipids.

The identity of the SM used is not critical for success. Thus, as used herein, the expression "SM" includes sphingomyelins derived or obtained from natural sources, as well as analogs and derivatives of naturally occurring SMs that are impervious to hydrolysis by LCAT, as is naturally occurring SM. SM is a phospholipid very similar in structure to lecithin, but, unlike lecithin, it does not have a glycerol backbone, and hence does not have ester linkages attaching the acyl chains. Rather, SM has a ceramide backbone, with amide linkages connecting the acyl chains. The SM can be obtained from virtually any source. For example, the SM can be obtained from milk, egg or brain. SM analogues or derivatives can also be used. Non-limiting examples of useful SM analogues and derivatives include, but are not limited to, palmitoylsphingomyelin, N-palmitoyl-4-hydroxysphinganine-1-phosphocholine (a form of phytosphingomyelin), palmitoylsphingomyelin, stearoylsphingomyelin, D-erythro-N-16:0-sphingomyelin and its dihydro isomer, D-erythro-N-16:0-dihydro-sphingomyelin. Synthetic SM such as synthetic palmitoylsphingomyelin or N-palmitoyl-4-hydroxysphinganine-1-phosphocholine (phytosphingomyelin) can be used in order to produce more homogeneous complexes and with fewer contaminants and/or oxidation products than sphingolipids of animal origin. Methods for synthesizing SM are described in U.S. Publication No. 2016/0075634.

Sphingomyelins isolated from natural sources can be artificially enriched in one particular saturated or unsaturated acyl chain. For example, milk sphingomyelin (Avanti Phospholipid, Alabaster, Ala.) is characterized by long saturated acyl chains (*i*.*e*., acyl chains having 20 or more carbon atoms). In contrast, egg sphingomyelin is characterized by short saturated acyl chains (i.e., acyl chains having fewer than 20 carbon atoms). For example, whereas only about 20% of milk sphingomyelin comprises C16:0 (16 carbon, saturated) acyl chains, about 80% of egg sphingomyelin comprises C16:0 acyl chains. Using solvent extraction, the composition of milk sphingomyelin can be enriched to have an acyl chain composition comparable to that of egg sphingomyelin, or vice versa.

The SM can be semi-synthetic such that it has particular acyl chains. For example, milk sphingomyelin can be first purified from milk, then one particular acyl chain, *e*.*g*., the C16:0 acyl chain, can be cleaved and replaced by another acyl chain. The SM can also be entirely synthesized, by *e.g.,* large-scale synthesis. See, *e.g.,* Dong et al., U.S. Pat. No. 5,220,043, entitled Synthesis of D-erythro-sphingomyelins, issued Jun. 15, 1993; Weis, 1999, Chem. Phys. Lipids 102 (1-2):3-12. SM can be fully synthetic, *e*.*g*., as described in U.S. Publication No. 2014/0275590.

The lengths and saturation levels of the acyl chains comprising a semi-synthetic or a synthetic SM can be selectively varied. The acyl chains can be saturated or unsaturated, and can contain from about 6 to about 24 carbon atoms. Each chain can contain the same number of carbon atoms or, alternatively each chain can contain different numbers of carbon atoms. In some embodiments, the semi-synthetic or synthetic SM comprises mixed acyl chains such that one chain is saturated and one chain is unsaturated. In such mixed acyl chain SMs, the chain lengths can be the same or different. In other embodiments, the acyl chains of the semi-synthetic or synthetic SM are either both saturated or both unsaturated. Again, the chains can contain the same or different numbers of carbon atoms. In some embodiments, both acyl chains comprising the semi-synthetic or synthetic SM are identical. In a specific embodiment, the chains correspond to the acyl chains of a naturally-occurring fatty acid, such as for example oleic, palmitic or stearic acid. In another embodiment, SM with saturated or unsaturated functionalized chains is used. In another specific embodiment, both acyl chains are saturated and contain from 6 to 24 carbon atoms. Non-limiting examples of acyl chains present in commonly occurring fatty acids that can be included in semi-synthetic and synthetic SMs are provided in Table 1, above.

In some embodiments, the SM is palmitoyl SM, such as synthetic palmitoyl SM, which has C16:0 acyl chains, or is egg SM, which includes as a principal component palmitoyl SM.

In a specific embodiment, functionalized SM, such as phytosphingomyelin, is used.

Lecithin can be derived or isolated from natural sources, or it can be obtained synthetically. Examples of suitable lecithins isolated from natural sources include, but are not limited to, egg phosphatidylcholine and soybean phosphatidylcholine. Additional non-limiting examples of suitable lecithins include, dipalmitoylphosphatidylcholine, dimyristoylphosphatidylcholine, distearoylphosphatidylcholine 1-myristoy1-2-palmitoylphosphatidylcholine, 1-palmitoy1-2-myristoylphosphatidylcholine, 1-palmitoyl-2-stearoylphosphatidylcholine, 1-stearoyl-2-palmitoylphosphatidylcholine, 1-palmitoyl-2-oleoylphosphatidylcholine, 1-oleoy1-2-palmitylphosphatidylcholine, dioleoylphosphatidylcholine and the ether derivatives or analogs thereof.

Lecithins derived or isolated from natural sources can be enriched to include specified acyl chains. In embodiments employing semi-synthetic or synthetic lecithins, the identity(ies) of the acyl chains can be selectively varied, as discussed above in connection with SM. In some embodiments of the Apomers described herein, both acyl chains on the lecithin are identical. In some embodiments of Apomers that include both SM and lecithin, the acyl chains of the SM and lecithin are all identical. In a specific embodiment, the acyl chains correspond to the acyl chains of myristitic, palmitic, oleic or stearic acid.

The Apomers preferably include one or more negatively charged phospholipids (*e*.*g*., alone or in combination with one or more neutral phospholipids). As used herein, "negatively charged phospholipids" are phospholipids that have a net negative charge at physiological pH. The negatively charged phospholipid can comprise a single type of negatively charged phospholipid, or a mixture of two or more different, negatively charged, phospholipids. In some embodiments, the charged phospholipids are negatively charged glycerophospholipids. Specific examples of suitable negatively charged phospholipids include, but are not limited to, a 1,2-dipalmitoyl-*sn*-glycero-3-[phospho-*rac*-(1-glycerol)], a phosphatidylglycerol, a phospatidylinositol, a phosphatidylserine, a phosphatidic acid, and salts thereof (*e*.*g*., sodium salts or potassium salts). In some embodiments, the negatively charged phospholipid comprises one or more of phosphatidylinositol, phosphatidylserine, phosphatidylglycerol and/or phosphatidic acid. In a specific embodiment, the negatively charged phospholipid comprises or consists of a salt of a phosphatidylglycerol or a salt of a phosphatidylinositol. In another specific embodiment, the negatively charged phospholipid comprises or consists of 1,2-dipalmitoyl-*sn*-glycero-3-[phospho-*rac*-(1-glycerol)], or DPPG, or a salt thereof.

The negatively charged phospholipids can be obtained from natural sources or prepared by chemical synthesis. In embodiments employing synthetic negatively charged phospholipids, the identities of the acyl chains can be selectively varied, as discussed above in connection with SM. In some embodiments of the Apomers described herein, both acyl chains on the negatively charged phospholipids are identical. In some embodiments, the acyl chains all types of phospholipids included in an Apomer are all identical. In a specific embodiment, the Apomer comprises negatively charged phospholipid(s), and/or SM all having C16:0 or C16:1 acyl chains. In a specific embodiment the fatty acid moiety of the SM is predominantly C16:1 palmitoyl. In one specific embodiment, the acyl chains of the charged phospholipid(s), lecithin and/or SM correspond to the acyl chain of palmitic acid. In yet another specific embodiment, the acyl chains of the charged phospholipid(s), lecithin and/or SM correspond to the acyl chain of oleic acid.

Apomers can include one or more positively charged lipids (*e*.*g*., alone or in combination with one or more neutral phospholipids). Examples of positively charged phospholipids that can be included in the Apomers of the disclosure include N1-[2-((1S)-1-[(3-aminopropyl)amino]-4-[di(3-amino-propyl)amino]butylcarboxamido)ethyl]-3,4-di[oleyloxy]-benzamide, 1,2-di-O-octadecenyl-3-trimethylammonium propane, 1,2-dimyristoleoyl-sn-glycero-3-ethylphosphocholine, 1-palmitoyl-2-oleoyl-sn-glycero-3-ethylphosphocholine, 1,2-dioleoyl-sn-glycero-3-ethylphosphocholine, 1,2-distearoyl-sn-glycero-3-ethylphosphocholine, 1,2-dipalmitoyl-sn-glycero-3-ethylphosphocholine, 1,2-dimyristoyl-sn-glycero-3-ethylphosphocholine, 1,2-dilauroyl-sn-glycero-3-ethylphosphocholine, 1,2-dilauroyl-sn-glycero-3-ethylphosphocholine, 1,2-dioleoyl-3-dimethylammonium-propane1,2-dimyristoyl-3-dimethylammonium-propane, 1,2-dipalmitoyl-3-dimethylammonium-propane, N-(4-carboxybenzyl)-N,N-dimethyl-2,3-bis(oleoyloxy)propan-1-aminium, 1,2-dioleoyl-3-trimethylammonium-propane, 1,2-dioleoyl-3-trimethylammonium-propane, 1,2-stearoyl-3-trimethylammonium-propane, 1,2-dipalmitoyl-3-trimethylammonium-propane, 1,2-dimyristoyl-3-trimethylammonium-propane, N-[1-(2,3-dimyristyloxy)propyl]-N, N-dimethyl-N-(2-hydroxyethyl) ammonium bromide, N,N,N-trimethyl-2-bis[(1-oxo-9-octadecenyl)oxy]-(Z,Z)- 1propanaminium methyl sulfate, and salts thereof (*e*.*g*., chloride or bromide salts). Other positively charged lipids such as stearylamine can also be used.

The lipids used are preferably at least 95% pure, and/or have reduced levels of oxidative agents (such as but not limited to peroxides). Lipids obtained from natural sources preferably have fewer polyunsaturated fatty acid moieties and/or fatty acid moieties that are not susceptible to oxidation. The level of oxidation in a sample can be determined using an iodometric method, which provides a peroxide value, expressed in milli-equivalent number of isolated iodines per kg of sample, abbreviated meq O/kg. *See, e*.*g*., Gray, 1978, Measurement of Lipid Oxidation: A Review, Journal of the American Oil Chemists Society 55:539-545; Heaton, F.W. and Ur, Improved iodometric Methods for the Determination of Lipid Peroxides, 1958, Journal of the Science of Food and Agriculture 9:781-786. Preferably, the level of oxidation, or peroxide level, is low, *e*.*g*., less than 5 meq O/kg, less than 4 meq O/kg, less than 3 meq O/kg, or less than 2 meq O/kg.

Apomers can in some embodiments include small quantities of additional lipids. Virtually any type of lipids can be used, including, but not limited to, lysophospholipids, galactocerebroside, gangliosides, cerebrosides, glycerides, triglycerides, and sterols and sterol derivatives (*e*.*g*., a plant sterol, an animal sterol, such as cholesterol, or a sterol derivative, such as a cholesterol derivative). For example, an Apomer can contain cholesterol or a cholesterol derivative, *e*.*g*., a cholesterol ester. The cholesterol derivative can also be a substituted cholesterol or a substituted cholesterol ester. The Apomers of the disclosure can also contain an oxidized sterol such as, but not limited to, oxidized cholesterol or an oxidized sterol derivative (such as, but not limited to, an oxidized cholesterol ester).. In some embodiments, the Apomers do not include cholesterol and/or its derivatives (such as a cholesterol ester or an oxidized cholesterol ester).

The lipid molecules (*e*.*g*., phospholipid molecules) can together contribute a net charge of 1-3 (*e.g.,* 1-3, 1-2, 2-3, 1, 2, or 3) per apolipoprotein molecule in the Apomer. In some embodiments, the net charge is negative. In other embodiments, the net charge is positive.

### 6.1.2.2. Detergents

The Apomers of the disclosure can contain one or more detergents. The detergent can be zwitterionic, nonionic, cationic, anionic, or a combination thereof. Exemplary zwitterionic detergents include 3-[(3-Cholamidopropyl)dimethylammonio]-1-propanesulfonate (CHAPS), 3-[(3-Cholamidopropyl)dimethylammonio]-2-hydroxy-1-propanesulfonate (CHAPSO), and N,N-dimethyldodecylamine N-oxide (LDAO). Exemplary nonionic detergents include D-(+)-trehalose 6-monooleate, N-octanoyl-N-methylglucamine, N-nonanoyl-N-methylglucamine, N-decanoyl-N-methylglucamine, 1-(7Z-hexadecenoyl)-*rac*-glycerol, 1-(8Z-hexadecenoyl)-*rac*-glycerol, 1-(8Z-heptadecenoyl)-*rac-*glycerol, 1-(9Z-hexadecenoyl)-*rac*-glycerol, 1-decanoyl-*rac*-glycerol. Exemplary cationic detergents include (S)-O-methyl-serine dodecylamide hydrochloride, dodecylammonium chloride, decyltrimethylammonium bromide, and cetyltrimethylammonium sulfate. Exemplary anionic detergents include cholesteryl hemisuccinate, cholate, alkyl sulfates, and alkyl sulfonates.

In some embodiments, the Apomers of the disclosure lack detergents.

### 6.1.2.3. Fatty acids

The Apomers can contain one or more fatty acids. The one or more fatty acids can include short-chain fatty acids having aliphatic tails of five or fewer carbons (*e*.*g*. butyric acid, isobutyric acid, valeric acid, or isovaleric acid), medium-chain fatty acids having aliphatic tails of 6 to 12 carbons (*e*.*g*., caproic acid, caprylic acid, capric acid, or lauric acid), long-chain fatty acids having aliphatic tails of 13 to 21 carbons (*e*.*g*., myristic acid, palmitic acid, stearic acid, or arachidic acid), very long chain fatty acids having aliphatic tails of 22 or more carbons (*e*.*g*., behenic acid, lignoceric acid, or cerotic acid), or a combination thereof. The one or more fatty acids can be saturated (*e*.*g*., caprylic acid, capric acid, lauric acid, myristic acid, palmitic acid, stearic acid, arachidic acid, behenic acid, lignoceric acid, or cerotic acid), unsaturated (*e*.*g*., myristoleic acid, palmitoleic acid, sapienic acid, oleic acid, elaidic acid, vaccenic acid, linoleic acid, linoelaidic acid, α-linolenic acid, arachidonic acid, eicosapentaenoic acid, erucic acid, or docosahexaenoic acid) or a combination thereof. Unsaturated fatty acids can be cis or trans fatty acids. In some embodiments, unsaturated fatty acids used in the Apomers of the disclosure are cis fatty acids.

### 6.1.2.4. Apolar molecules and sterols attached to a sugar

The Apomers can contain one or more amphipathic molecules that comprise an apolar molecule or moiety (*e*.*g*., a hydrocarbon chain, an acyl or diacyl chain) or a sterol (e.g., cholesterol) attached to a sugar (*e*.*g*., a monosaccharide such as glucose or galactose, or a disaccharide such as maltose or trehalose). The sugar can be a modified sugar or a substituted sugar. Exemplary amphipathic molecules comprising an apolar molecule attached to a sugar include dodecan-2-yloxy-β-D-maltoside, tridecan-3-yloxy-β-D-maltoside, tridecan-2-yloxy-β-D-maltoside, n-dodecyl-β-D-maltoside (DDM), n-octyl-β-D-glucoside, n-nonyl-β-D-glucoside, n-decyl-β-D-maltoside, n-dodecyl-β-D-maltopyranoside, 4-n-Dodecyl-α,α-trehalose, 6-n-dodecyl-α,α-trehalose, and 3-n-dodecyl-α,α-trehalose.

### 6.2. Compositions comprising Apomers

The disclosure provides compositions comprising a population of Apomers of the disclosure.

Compositions of the disclosure can comprise populations of Apomers having different average apolipoprotein to amphipathic molecule ratios. For example, a population comprising apolipoprotein and negatively charged amphipathic molecules (*e*.*g*., negatively charged phospholipids) can have an average of 1.8 to 2.5 apolipoprotein molecules (*e.g.,* about 2) and 0.9 to 2.5 negatively charged amphipathic molecules (*e.g.,* about 1 or about 2). As another example, a population comprising apolipoprotein and negatively charged amphipathic molecules (*e*.*g*., negatively charged phospholipids) can have an average of 3.5 to 4.5 apolipoprotein molecules (*e*.*g*., about 4) and 0.9 to 2.5 negatively charged amphipathic molecules (*e.g*., about 1 or about 2). As another example, a population comprising apolipoprotein and negatively charged amphipathic molecules (*e*.*g*., negatively charged phospholipids) can have an average of 7 to 9 apolipoprotein molecules (*e.g.,* about 8) and 0.9 to 2.5 negatively charged amphipathic molecules (*e.g.,* about 1 or about 2).

The identity and amount of lipoprotein molecules in a composition of Apomers can be determined, for example, by mass spectrometry (see, *e*.*g*., Zhang et al., 2010, Methods Mol Biol. 673: 211-222). The identity and amount of amphipathic molecules in a composition of Apomers can be determined, for example, by thin layer chromatography (see, *e*.*g*., Clogston and Patri, 2011, Methods Mol Biol. 697:109-17). The presence of discoidal particles in a composition of Apomers can be determined, for example, using NMR spectroscopy, atomic force microscopy, electron microscopy, or other suitable technique known in the art.

Preferably, the compositions contain, if at all, only a small amount of aggregated apolipoprotein. For example, in some embodiments, no more than 10% of the apolipoprotein molecules in the composition are in aggregate form. In other embodiments, no more than 5% of the apolipoprotein molecules in the composition are in aggregate form. In yet other embodiments, no more than 2% of the apolipoprotein molecules in the composition are in aggregate form. In yet other embodiments, aggregates of apolipoprotein are undetectable, for example when measured using gel permeation chromatography.

In some embodiments, the Apomers in a composition primarily comprise multimeric apolipoprotein molecules. In some embodiments, no more than 20% of the apolipoprotein molecules in the composition are in monomeric form. In other embodiments, no more than 10% of the apolipoprotein molecules in the composition are in monomeric form. In yet other embodiments, no more than 5% of the apolipoprotein molecules in the composition are in monomeric form. In yet other embodiments, no more than 2% of the apolipoprotein molecules in the composition are in monomeric form.

Preferably, the compositions of the disclosure contain only a small amount of uncomplexed amphipathic molecules. In some embodiments, no more than 20% of the amphipathic molecules in the composition are in uncomplexed form. In other embodiments, no more than 10% of the amphipathic molecules are in uncomplexed form. In yet other embodiments, no more than 5% of the amphipathic molecules are uncomplexed form. In yet other embodiments, no more than 2% of the amphipathic molecules are in uncomplexed form.

The homogeneity of the Apomers and compositions of the disclosures can be measured by gel permeation chromatography. A highly homogeneous composition will generally have a main peak corresponding to the Apomers and, possibly, one or more secondary peaks corresponding to one or more of free protein and free amphipathic molecules. Secondary peaks corresponding to Apomers or complexes having a different size from the Apomers in the main peak may also be seen. The area of the main peak on a gel permeation chromatogram relative to the total area of the main and secondary peaks determines the percent homogeneity of a composition. In some embodiments, the compositions of the disclosure are at least 75% homogeneous. In other embodiments, the compositions of the disclosure are at least 85% homogeneous. In other embodiments, the compositions of the disclosure are at least 95% homogeneous. In yet other embodiments, the compositions of the disclosure are at least 98% homogeneous.

In some embodiments, the homogeneity of a population of Apomers in a composition (*i*.*e*., the area of the main peak relative to the total area of the main and secondary peaks corresponding to Apomers) is at least 75%. In other embodiments, the population is at least 85% homogeneous. In other embodiments, the population is at least 95% homogeneous. In other embodiments, the population is at least 98% homogeneous.

Compositions of the disclosure comprising populations of Apomers can be further characterized by analyzing the Stokes radii of particles in a given composition (*e*.*g*., by gel filtration chromatography). Preferably, the compositions of the disclosure contain, if any, only a small amount of discoidal lipoprotein complexes. In some embodiments, lipoprotein complexes having Stokes radii of greater than 3.4 nm, if present, represent no more than 10% of the apolipoprotein in a composition on a weight basis. In other embodiments, lipoprotein complexes having Stokes radii of greater than 3.4 nm, if present, represent no more than 5% of the apolipoprotein in a composition on a weight basis. In yet other embodiments, lipoprotein complexes having Stokes radii of greater than 3.4 nm, if present, represent no more than 2% of the apolipoprotein in a composition on a weight basis.

In some embodiments, at least 75% of the particles in a composition have a Stokes radius of less than 3.5 nm. In other embodiments, at least 85% of the particles in a composition have a Stokes radius of less than 3.5 nm. In other embodiments, at least 95% of the particles in a population have a Stokes radius of less than 3.5 nm. In other embodiments, at least 98% of the particles in a population have a Stokes radius of less than 3.5 nm.

An Apomer composition of the disclosure can be in the form of a pharmaceutical composition. Pharmaceutical compositions can include a population of Apomers and one or more pharmaceutically acceptable carriers, excipients, diluents, or a combination thereof.

Exemplary carriers include solvents or dispersion media containing, for example, water, ethanol, a polyol (for example, glycerol, propylene glycol, and liquid polyethylene glycol, and the like), suitable mixtures thereof, and vegetable oils. Exemplary diluents include water for injection, saline solution, buffered solutions such as phosphate buffered saline solution, and sugar solutions such as sucrose or dextran solutions. Exemplary excipients include fillers, binders, disintegrants, solvents, solubilizing agents, and coloring agents.

The compositions of the disclosure can be formulated according to techniques known in the art (*e.g.,* as described in Allen et al., eds., 2012, Remington: The Science and Practice of Pharmacy, 22nd Edition, Pharmaceutical Press, London, UK). For example, the compositions can be formulated for subcutaneous, intradermal, intravenous, or intraperitoneal injection (*e*.*g*., as a solution), inhalation (intranasal or intrapulmonary inhalation), implantation (*e.g.,* as a suppository), ocular or intraocular administration (*e.g.,* via eye drops).

In some embodiments, the compositions are packaged in unit dosage amounts suitable for administration. For example, in some embodiments, the compositions comprise unit dosage amounts of dried (for example lyophilized) Apomers packaged in sealed vials. Such compositions are suitable for reconstitution with water, physiological solution (such as saline) or buffer, and administration via injection. Such compositions may optionally include one or more anti-caking and/or anti-agglomerating agents to facilitate reconstitution of the Apomers, or one or more buffering agents, isotonicity agents (e.g., sucrose and/or mannitol), sugars or salts (e.g., sodium chloride) designed to adjust the pH, osmolality and/or salinity of the reconstituted suspension. The compositions described above can be manufactured under conditions that minimize oxidation, thereby reducing the risk of side effects, such as liver damage, caused by oxidized products. For example, the compositions can be manufactured under an inert gas, such as nitrogen, helium, or argon.

Apomers may also be formulated in pharmaceutical compositions for controlled release. As used herein, "controlled release" refers to release of an Apomer from a formulation at a rate that the blood concentration of the Apomer in an individual is maintained within the therapeutic range for an extended duration, over a time period on the order of hours, days, weeks, or longer. Apomers may be formulated in a bioerodible or nonbioerodible controlled matrix, a number of which are well known in the art. A controlled release matrix may include a synthetic polymer or copolymer, for example in the form of a hydrogel. Examples of such polymers include polyesters, polyorthoesters, polyanhydrides, polysaccharides, poly(phosphoesters), polyamides, polyurethanes, poly(imidocarbonates) and poly(phosphazenes), and poly-lactide-co-glycolide (PLGA), a copolymer of poly(lactic acid) and poly(glycolic acid). Collagen, albumin, and fibrinogen containing materials may also be used.

### 6.3. Uses of the Apomers

The Apomers and pharmaceutical compositions of the disclosure can be used to treat dyslipidemic disorders and hepatic disorders. The following uses and methods are to be construed as directed to the Apomers for use in said methods and uses.

Dyslipidemic disorders include, but are not limited to, hyperlipidemia or susceptibility to hyperlipidemia (such as hypercholesterolemia), cardiovascular disease or susceptibility to cardiovascular disease. Non-limiting examples of cardiovascular disease for which the Apomers and pharmaceutical compositions can be useful are atherosclerosis, stroke, myocardial infarction, acute coronary syndrome, angina pectoris, intermittent claudication, critical limb ischemia, atrial valve sclerosis and restenosis. Dyslipidemic disorders caused by genetic defects such as, but not limited to, ApoA-I deficiency, ABCA1 deficiency, and Familial Hypercholesterolemia in homozygote or heterozygote states can be treated with the Apomers and pharmaceutical compositions of the disclosure.

Non-limiting examples of hepatic disorders for which the Apomers and pharmaceutical compositions can be useful are non-alcoholic fatty liver disease (NAFLD), non-alcoholic steatohepatitis (NASH), and hepatitis, including viral hepatitis such as caused by infection with the hepatitis A, B or C viruses and non-viral hepatitis, for example resulting from the use of recreational or prescription drugs.

The methods of treatment can comprise administering a therapeutically effective amount of an Apomer or a pharmaceutical composition containing a therapeutically effective amount of the Apomer to the subject.

The Apomers and compositions can be administered in the methods of the disclosure to a subject (which is preferably a mammal and most preferably a human) by any suitable route. For example, administration can be via injection (*e*.*g*., subcutaneous, intradermal, intravenous, or intraperitoneal injection), inhalation (*e.g.,* intranasal or intrapulmonary inhalation, implantation, optionally (*e*.*g*., via a suppository), or ocular or intraocular routes (*e*.*g*., via eye drops). In some embodiments, the solution is administered as a depot injection.

The methods of the disclosure can further comprise adjunctively administering a second therapeutic agent to the subject. For example, the second therapeutic agent can comprise a bile-acid resin, niacin, an anti-inflammatory agent, a statin, a fibrate, a CETP inhibitor, a platelet aggregation inhibitor, an anticoagulant, an agonist of PCSK9, an inhibitor of cholesterol absorption, or any combination thereof (such as a combination of a statin and niacin or a combination of a statin and a cholesterol absorption inhibitor).

Exemplary bile-acid resins include cholestyramine, colestipol, and colesevelam.

Exemplary anti-inflammatory agents include Alclofenac; Alclometasone Dipropionate; Algestone Acetonide; Alpha Amylase; Amcinafal; Amcinafide; Amfenac Sodium; Amiprilose Hydrochloride; Anakinra; Anirolac; Anitrazafen; Apazone; Aspirin; Balsalazide Disodium; Bendazac; Benoxaprofen; Benzydamine Hydrochloride; Bromelains; Broperamole; Budesonide; Carprofen; Cicloprofen; Cintazone; Cliprofen; Clobetasol Propionate; Clobetasone Butyrate; Clopirac; Cloticasone Propionate; Cormethasone Acetate; Cortodoxone; Deflazacort; Desonide; Desoximetasone; Dexamethasone Dipropionate; Diclofenac Potassium; Diclofenac Sodium; Diflorasone Diacetate; Diflumidone Sodium; Diflunisal; Difluprednate; Diftalone; Dimethyl Sulfoxide; Drocinonide; Endrysone; Enlimomab; Enolicam Sodium; Epirizole; Etodolac; Etofenamate; Felbinac; Fenamole; Fenbufen; Fenclofenac; Fenclorac; Fendosal; Fenpipalone; Fentiazac; Flazalone; Fluazacort; Flufenamic Acid; Flumizole; Flunisolide Acetate; Flunixin; Flunixin Meglumine; Fluocortin Butyl; Fluorometholone Acetate; Fluquazone; Flurbiprofen; Fluretofen; Fluticasone Propionate; Furaprofen; Furobufen; Halcinonide; Halobetasol Propionate; Halopredone Acetate; Ibufenac; Ibuprofen; Ibuprofen Aluminum; Ibuprofen Piconol; Ilonidap; Indomethacin; Indomethacin Sodium; Indoprofen; Indoxole; Intrazole; Isoflupredone Acetate; Isoxepac; Isoxicam; Ketoprofen; Lofemizole Hydrochloride; Lornoxicam; Loteprednol Etabonate; Meclofenamate Sodium; Meclofenamic Acid; Meclorisone Dibutyrate; Mefenamic Acid; Mesalamine; Meseclazone; Methylprednisolone Suleptanate; Morniflumate; Nabumetone; Naproxen; Naproxen Sodium; Naproxol; Nimazone; Olsalazine Sodium; Orgotein; Orpanoxin; Oxaprozin; Oxyphenbutazone; Paranyline Hydrochloride; Pentosan Polysulfate Sodium; Phenbutazone Sodium Glycerate; Pirfenidone; Piroxicam; Piroxicam Cinnamate; Piroxicam Olamine; Pirprofen; Prednazate; Prifelone; Prodolic Acid; Proquazone; Proxazole; Proxazole Citrate; Rimexolone; Romazarit; Salcolex; Salnacedin; Salsalate; Salycilates; Sanguinarium Chloride; Seclazone; Sermetacin; Sudoxicam; Sulindac; Suprofen; Talmetacin; Talniflumate; Talosalate; Tebufelone; Tenidap; Tenidap Sodium; Tenoxicam; Tesicam; Tesimide; Tetrydamine; Tiopinac; Tixocortol Pivalate; Tolmetin; Tolmetin Sodium; Triclonide; Triflumidate; Zidometacin; Glucocorticoids; and Zomepirac Sodium. In some embodiments, the anti-inflammatory agent comprises aspirin.

Exemplary statins include atorvastatin, rosuvastatin, pravastatin, simvastatin, and lovastatin. In some embodiments, the statin comprises atorvastatin. In other embodiments, the statin comprises rosuvastatin. In other embodiments, the statin comprises pravastatin. In other embodiments, the statin comprises simvastatin. In other embodiments, the statin comprises lovastatin.

Exemplary fibrates include fenofibrate, bezafibrate, clinofibrate, and gemfibrozil. In some embodiments, the fibrate comprises fenofibrate. In some embodiments, the fibrate comprises bezafibrate. In some embodiments, the fibrate comprises clinofibrate. In some embodiments, the fibrate comprises gemfibrozil.

Exemplary CETP inhibitors include anacetrapib, dalcetrapib, and evacetrapib.

Exemplary platelet aggregation inhibitors include irreversible cyclooxygenase (COX) inhibitors (*e.g.,* aspirin) and adenosine diphosphate (ADP) receptor inhibitors (*e.g.,* clopidogrel, prasugrel, ticagrelor, and ticlopidine).

Exemplary anticoagulants include coumarins (*e*.*g*., warfarin, acenocoumarol, phenprocoumon, atromentin, phenindione), heparins (*e*.*g*., unfractionated heparin and low molecular weight (LMW) heparins such as enoxaparin, nadroparin and dalteparin), direct factor Xa inhibitors (*e*.*g*., apixaban, rivaroxaban, dabigatran, and edoxaban), omega-3 fatty acids, omega-3 fatty acid ethyl esters, and combinations thereof. In some embodiments, the anticoagulant comprises aspirin.

Exemplary antagonists of PCSK9 include antibodies such as alirocumab, bococizumabevolocumab, 1D05-IgG2 (Ni et al., 2011, J Lipid Res. 52(1):78-86), and LY3015014 (Kastelein et al., 2016, Eur Heart J 37(17):1360-9) and an RNAi therapeutics such as ALN-PCSSC (the Medicines Company)).

Exemplary inhibitors of cholesterol absorption include ezetimibe and clopidogrel bisulfate. In some embodiments, the inhibitor of cholesterol absorption comprises ezetimibe. In other embodiments, the inhibitor of cholesterol absorption comprises clopidogrel (*e*.*g*., clopidogrel bisulfate).

Other lipid modulators, such as gemcabene (developed by Gemphire Therapeutics) and bempedoic acid (developed by Esperion Therapeutics) can also be adjunctively administered with an Apomer of the disclosure.

Unless required otherwise by context, identification of a specific agent encompasses salts thereof. Thus, for example, recitation of "warfarin" encompasses "warfarin sodium," recitation of "clopidogrel" encompasses "clopidogrel bisulfate," *etc.*

### 7. EXAMPLES

### 7.1. Example 1: ApoA-I/DPPG/Sphingomyelin Apomers

Equimolar amounts of 1,2-dipalmitoyl-*sn*-glycero-3-[phospho-*rac*-(1-glycerol)] (DPPG) and sphingomyelin (SM) were weighed, solubilized in CHCl₃ and dried under a stream of N₂. The lipid film was dispersed in 10 mM phosphate buffer pH 8.0 at 37°C using a high sheer Ultra-Turax T25 mixer for 5 min. The solution was kept under a nitrogen overlay during preparation.

A solution of purified human ApoA-I was thawed at room temperature. The ApoA-I solution and DPPG:SM solution were then mixed to provide a mixture having ApoA-I, DPPG, and SM at a 1:2:2 molar ratio. The mixture was heated to 37°C for 4 hours. The mixture was then subjected to thermal cycling (three cycles of 20 minutes at 57°+/-2 °C and 5 minutes at 37° +/- 2 °C) to complete formation of the Apomers. After completion of the thermal cycling, the Apomer solution was cooled down and stored overnight at 4°C.

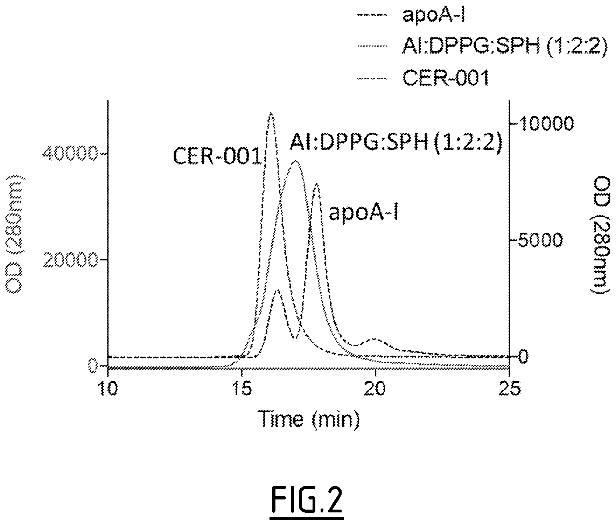

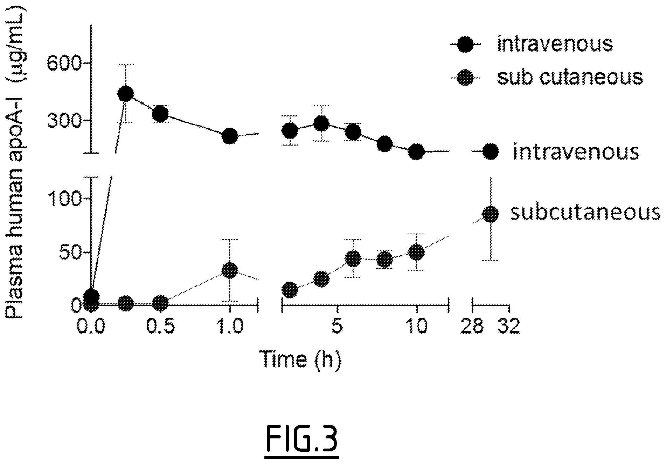

The Apomer solution was analyzed by gel permeation chromatography. As shown in Figure 2, the Apomers were smaller than discoidal CER-001, which comprises ApoA-I, SM, and DPPG in a 1:2.7 protein:lipid weight ratio with SM:DPPG weight ratio of 97:3. The Apomer solution was administered to rabbits by intravenous and sub-cutaneous injection. Plasma human ApoA-I levels following the administrations are shown in Figure 3.

### 8. SEQUENCE LISTING

| SEQ ID NO | Sequence |
|---|---|
| 1 | |
| 2 | PPQSPWDRVK DLATVYVDVL KDSGRDYVSQ FEGSALGKQL NLKLLDNWDS VTSTFSKLRE QLGPVTQEFW DNLEKETEGL RQEMSKDLEE VKAKVQPYLD DFQKKWQEEM ELYRQKVEPL RAELQEGARQ KLHELQEKLS PLGEEMRDRA RAHVDALRTH LAPYSDELRQ RLAARLEALK ENGGARLAEY HAKATEHLST LSEKAKPALE DLRQGLLPVL ESFKVSFLSA LEEYTKKLNT Q |

## Claims

1. A composition comprising a population of Apomers for use as a medicament, each Apomer comprising 1-8 apolipoprotein molecules complexed with amphipathic molecules, wherein:
(i) the amphipathic molecules contribute a net charge of at least +1 or -1 per molecule of apolipoprotein; and
(ii) the molar ratio of apolipoprotein molecules to amphipathic molecules ranges from 8:1 to 1:15.

2. The composition for use of claim 1, wherein the apolipoprotein to amphipathic molecule molar ratio ranges from 6:1 to 1:6.

3. The composition for use of claim 1 or claim 2, wherein the amphipathic molecules comprise a phospholipid, a detergent, a fatty acid, an apolar molecule or sterol covalently attached to a sugar, or a combination thereof, optionally wherein:
(a) the apolar molecule is an acyl or a diacyl chain; and/or
(b) the sugar is a modified sugar or a substituted sugar.

4. The composition for use of claim 3, wherein the amphipathic molecules comprise or consist of phospholipid molecules, optionally wherein the phospholipid molecules comprise negatively charged phospholipids, neutral phospholipids or a combination thereof, and further optionally, the phospholipid molecules contribute a net charge of 1-3 per apolipoprotein molecule in the Apomer.

5. The composition for use of claim 4, wherein the phospholipid molecules consist of a combination of negatively charged and neutral phospholipids, optionally wherein the molar ratio of negatively charge phospholipid to neutral phospholipid ranges from 1:1 to 1:3, further optionally about 1:1 or about 1:2.

6. The composition for use of any one of claims claim 1 to 5, wherein no more than 20%, no more than 10%, nor more than 5%, or no more than 2% of the apolipoprotein molecules in the composition are in aggregate form and/or no more than 20%, no more than 10%, no more than 5%, or no more than 2% of the apolipoprotein molecules in the composition are in monomeric form.

7. The composition for use of any one of claims 1 to 6, wherein at least 75%, at least 85%, at least 95%, or at least 98% of the particles in the population have a Stokes radius of less than 3.5 nm

8. The composition for use of any one of claims 1 to 7, wherein:
(a) Apomers in the population have on average 1.8 to 2.5 apolipoprotein molecules and 0.9-2.5 negatively charged amphipathic molecules, optionally about 2 apolipoprotein molecules and about 1 or about 2 negatively charged amphipathic molecules;
(b) Apomers in the population have on average 3.5 to 4.5 apolipoprotein molecules and 0.9-2.5 negatively charged amphipathic molecules, optionally about 4 apolipoprotein molecules and about 1 or about 2 negatively charged amphipathic molecules; or
(c) Apomers in the population have on average 7 to 9 apolipoprotein molecules and 0.9-2.5 negatively charged amphipathic molecules, optionally about 8 apolipoprotein molecules and about 1 or about 2 negatively charged amphipathic molecules.

9. The composition for use of any one of claims 1 to 8, wherein:
(a) no more than 20%, no more than 10%, no more than 5%, or no more than 2% of the amphipathic molecules are in uncomplexed form;
(b) the Apomers in the composition are at least 75%, at least 85%, at least 95%, or at least 98% homogeneous; and/or
(c) lipoprotein complexes having Stokes radii of greater than 3.4 nm, if present, represent no more than 10%, no more than 5%, or no more than 2% of the apolipoprotein in the composition on a weight basis.

10. The composition for use of any one of claims 1 to 9, wherein:
(a) the apolipoprotein molecules comprise or consist of apolipoprotein A-I (ApoA-I) molecules, which are optionally human ApoA-I molecules, which are optionally recombinant, optionally wherein:
(i) the ApoA-I molecules are Apolipoprotein A-I_{Miano} (ApoA-I_{M}), Apolipoprotein A-I_{Paris} (ApoA-I_{P}), or Apolipoprotein A-I_{Zaragoza} (ApoA-I_{Z}) molecules; and/or
(ii) the ApoA-I molecules comprise an amino acid sequence having at least 90%, at least 95%, at least 98%, or at least 99% sequence identity to SEQ ID NO:2; or
(b) the apolipoprotein molecules are ApoA-II, ApoA-IV, ApoA-V, ApoB, ApoC-I, ApoC-II, ApoC-III, ApoD, ApoE, ApoJ or ApoH molecules.

11. The composition for use of any one of claims 1 to 10, wherein the amphipathic molecules comprise a negatively charged phospholipid which optionally comprises a salt of a phosphatidylinositol, a phosphatidylserine, a phosphatidylglycerol or a phosphatidic acid, optionally wherein the salt is a sodium salt or a potassium salt, optionally wherein the negatively charged phospholipid is a salt of a phosphatidylglycerol, which is optionally a salt of 1,2-dipalmitoyl-sn-glycero-3-[phospho-rac-(1-glycerol)] (DPPG).

12. The composition for use of any one of claims 1 to 11, wherein the Apomers comprise a neutral phospholipid, which is optionally a lecithin or a sphingomyelin, optionally wherein the sphingomyelin is egg sphingomyelin, a plant sphingomyelin, or a synthetic sphingomyelin, optionally wherein the Apomers comprise a negatively charged phospholipid and a neutral phospholipid, wherein the molar ratio of negatively charge phospholipid to neutral phospholipid in the composition ranges from 1:1 to 1:3, optionally about 1:1 or about 1:2.

13. The composition for use of any one of claims 1 to 12 which is in the form of a pharmaceutical composition comprising one or more pharmaceutically acceptable carriers, diluents, and/or excipients.

14. The composition for use of any one of claims 1 to 13, for use in treating a dyslipidemic disorder in a subject in need thereof, optionally wherein the subject is human, optionally wherein:
(a) the subject has or is susceptible to hyperlipidemia or cardiovascular disease, optionally wherein said hyperlipidemia is hypercholesterolemia, and optionally wherein the cardiovascular disease is atherosclerosis, stroke, myocardial infarction, acute coronary syndrome, angina pectoris, intermittent claudication, critical limb ischemia, atrial valve sclerosis or restenosis; or
(b) the subject has an ApoA-I deficiency, an ABCA1 deficiency, or Familial Hypercholesterolemia.

15. The composition for use of any one of claims 1 to 13, for use in treating a liver disorder in a subject in need thereof, optionally wherein the subject is human, and optionally wherein said subject has non-alcoholic fatty liver disease (NAFLD), non-alcoholic steatohepatitis (NASH), or hepatitis.

## Patentansprüche

1. Zusammensetzung, umfassend eine Population von Apomeren zur Verwendung als ein Medikament, wobei jedes Apomer 1-8 Apolipoproteinmoleküle umfasst, die mit amphipathischen Molekülen komplexiert sind, wobei:
(i) die amphipathischen Moleküle eine Nettoladung von mindestens +1 oder -1 pro Molekül von Apolipoprotein beitragen; und
(ii) das Molverhältnis von Apolipoproteinmolekülen zu amphipathischen Molekülen im Bereich von 8:1 bis 1:15 liegt.

2. Zusammensetzung zur Verwendung nach Anspruch 1, wobei das Molverhältnis von Apolipoprotein zu amphipathischen Molekül im Bereich von 6:1 bis 1:6 liegt.

3. Zusammensetzung zur Verwendung nach Anspruch 1 oder Anspruch 2, wobei die amphipathischen Moleküle ein Phospholipid, ein Detergens, eine Fettsäure, ein apolares Molekül oder Sterol, das kovalent an einen Zucker gebunden ist, oder eine Kombination davon umfassen, wobei optional:
(a) das apolare Molekül eine Acyl- oder Diacylkette ist, und/oder
(b) der Zucker ein modifizierter Zucker oder ein substituierter Zucker ist.

4. Zusammensetzung zur Verwendung nach Anspruch 3, wobei die amphipathischen Moleküle Phospholipidmoleküle umfassen oder daraus bestehen, wobei optional die Phospholipidmoleküle negativ geladene Phospholipide, neutrale Phospholipide oder eine Kombination davon umfassen, und weiter optional die Phospholipidmoleküle eine Nettoladung von 1-3 pro Apolipoproteinmolekül im Apomer beitragen.

5. Zusammensetzung zur Verwendung nach Anspruch 4, wobei die Phospholipidmoleküle aus einer Kombination negativ geladener und neutraler Phospholipide bestehen, wobei optional das Molverhältnis von negativ geladenem Phospholipid zu neutralem Phospholipid im Bereich von 1:1 bis 1:3, weiter optional etwa 1:1 oder etwa 1:2 liegt.

6. Zusammensetzung zur Verwendung nach einem der Ansprüche 1 bis 5, wobei nicht mehr als 20 %, nicht mehr als 10 %, nicht mehr als 5 % oder nicht mehr als 2 % der Apolipoproteinmoleküle in der Zusammensetzung in aggregierter Form vorliegen und/oder nicht mehr als 20 %, nicht mehr als 10 %, nicht mehr als 5 %, oder nicht mehr als 2 % der Apolipoproteinmoleküle in der Zusammensetzung in monomerer Form vorliegen.

7. Zusammensetzung zur Verwendung nach einem der Ansprüche 1 bis 6, wobei mindestens 75 %, mindestens 85 %, mindestens 95 %, oder mindestens 98 % der Partikel in der Population einen Stokes-Radius von weniger als 3,5 nm aufweisen.

8. Zusammensetzung zur Verwendung nach einem der Ansprüche 1 bis 7, wobei:
(a) Apomere in der Population im Durchschnitt 1,8 bis 2,5 Apolipoproteinmoleküle und 0,9-2,5 negativ geladene amphipathische Moleküle aufweisen, optional etwa 2 Apolipoproteinmoleküle und etwa 1 oder etwa 2 negativ geladene amphipathische Moleküle;
(b) Apomere in der Population im Durchschnitt 3,5 bis 4,5 Apolipoproteinmoleküle und 0,9-2,5 negativ geladene amphipathische Moleküle aufweisen, optional etwa 4 Apolipoproteinmoleküle und etwa 1 oder etwa 2 negativ geladene amphipathische Moleküle; oder
(c) Apomere in der Population im Durchschnitt 7 bis 9 Apolipoproteinmoleküle und 0,9-2,5 negativ geladene amphipathische Moleküle aufweisen, optional etwa 8 Apolipoproteinmoleküle und etwa 1 oder etwa 2 negativ geladene amphipathische Moleküle.

9. Zusammensetzung zur Verwendung nach einem der Ansprüche 1 bis 8, wobei:
(a) nicht mehr als 20 %, nicht mehr als 10 %, nicht mehr als 5 %, oder nicht mehr als 2 % der amphipathischen Moleküle in unkomplexierter Form vorliegen;
(b) die Apomere in der Zusammensetzung zu mindestens 75 %, mindestens 85 %, mindestens 95 %, oder mindestens 98 % homogen sind, und/oder
(c) Lipoproteinkomplexe, die Stokes-Radien größer als 3,4 nm aufweisen, sofern vorhanden, nicht mehr als 10 %, nicht mehr als 5 %, oder nicht mehr als 2 % des Apolipoproteins in der Zusammensetzung auf Gewichtsbasis darstellen.

10. Zusammensetzung zur Verwendung nach einem der Ansprüche 1 bis 9, wobei:
(a) die Apolipoproteinmoleküle Apolipoprotein A-I (ApoA-I)-Moleküle umfassen oder daraus bestehen, die optional menschliche ApoA-I-Moleküle sind, die optional rekombinant sind, wobei optional:
(i) die ApoA-I-Moleküle Apolipoprotein A-I_{Milano} (ApoA-I_{M}), Apolipoprotein A-I_{Paris} (ApoA-I_{P}) oder Apolipoprotein A-I_{Zaragoza} (ApoA-I_{Z})-Moleküle sind, und/oder
(ii) die ApoA-I-Moleküle eine Aminosäuresequenz umfassen, die mindestens 90 %, mindestens 95 %, mindestens 98 %, oder mindestens 99 % Sequenzidentität mit SEQ ID NO: 2 aufweist; oder
(b) die Apolipoproteinmoleküle ApoA-II, ApoA-IV, ApoA-V, ApoB, ApoC-I, ApoC-II, ApoC-III, ApoD, ApoE, ApoJ oder ApoH-Moleküle sind.

11. Zusammensetzung zur Verwendung nach einem der Ansprüche 1 bis 10, wobei die amphipathischen Moleküle ein negativ geladenes Phospholipid umfassen, das optional ein Salz eines Phosphatidylinositols, eines Phosphatidylserins, eines Phosphatidylglycerins oder einer Phosphatidsäure umfasst, wobei optional das Salz ein Natriumsalz oder ein Kaliumsalz Salz ist, wobei optional das negativ geladene Phospholipid ein Salz eines Phosphatidylglycerins ist, das optional ein Salz von 1,2-Dipalmitoyl-sn-glycero-3-[phospho-rac-(1-glycerin)] (DPPG) ist.

12. Zusammensetzung zur Verwendung nach einem der Ansprüche 1 bis 11, wobei die Apomere ein neutrales Phospholipid umfassen, das optional ein Lecithin oder ein Sphingomyelin ist, wobei optional das Sphingomyelin Eier-Sphingomyelin, ein pflanzliches Sphingomyelin oder ein synthetisches Sphingomyelin ist, wobei optional die Apomere ein negativ geladenes Phospholipid und ein neutrales Phospholipid umfassen, wobei das Molverhältnis von negativ geladenem Phospholipid zu neutralem Phospholipid in der Zusammensetzung im Bereich von 1:1 bis 1:3, optional etwa 1:1 oder etwa 1:2 liegt.

13. Zusammensetzung zur Verwendung nach einem der Ansprüche 1 bis 12, die in der Form einer pharmazeutischen Zusammensetzung vorliegt, umfassend einen oder mehrere pharmazeutisch verträgliche Träger, Verdünnungsmittel und/oder Hilfsstoffe.

14. Zusammensetzung zur Verwendung nach einem der Ansprüche 1 bis 13, zur Verwendung bei der Behandlung einer dyslipidämischen Störung bei einem Subjekt, das diese benötigt, wobei optional das Subjekt ein Mensch ist, wobei optional:
(a) das Subjekt eine Hyperlipidämie oder eine Herz-Kreislauf-Erkrankung aufweist oder dafür anfällig ist, wobei optional die Hyperlipidämie Hypercholesterinämie ist, und wobei optional die Herz-Kreislauf-Erkrankung Atherosklerose, Schlaganfall, Myokardinfarkt, akutes Koronarsyndrom, Angina pectoris oder Claudicatio intermitens, kritische Gliedmaßenischämie, Vorhofklappensklerose oder -restenose ist; oder
(b) das Subjekt einen ApoA-I-Mangel, einen ABCA1-Mangel oder eine familiäre Hypercholesterinämie aufweist.

15. Zusammensetzung zur Verwendung nach einem der Ansprüche 1 bis 13, zur Verwendung bei der Behandlung einer Leberstörung bei einem Subjekt, das diese benötigt, wobei optional das Subjekt ein Mensch ist, und wobei optional das Subjekt eine nichtalkoholische Fettlebererkrankung (NAFLD), eine nichtalkoholische Steatohepatitis (NASH) oder Hepatitis aufweist.

## Revendications

1. Composition comprenant une population d'apomères pour utilisation en tant que médicament, chaque apomère comprenant 1-8 molécules d'apolipoprotéine complexées avec des molécules amphipathiques, dans laquelle :
(i) les molécules amphipathiques contribuent à une charge nette d'au moins +1 ou -1 par molécule d'apolipoprotéine ; et
(ii) le rapport molaire des molécules d'apolipoprotéine aux molécules amphipathiques va de 8:1 à 1:15.

2. Composition pour utilisation selon la revendication 1, dans laquelle le rapport molaire des molécules d'apolipoprotéine aux molécules amphipathiques va de 6:1 à 1:6.

3. Composition pour utilisation selon la revendication 1 ou la revendication 2, dans laquelle les molécules amphipathiques comprennent un phospholipide, un détergent, un acide gras, une molécule apolaire ou un stérol fixé de manière covalente à un sucre, ou une combinaison de ceux-ci, éventuellement dans laquelle :
(a) la molécule apolaire est une chaîne acyle ou diacyle ; et/ou
(b) le sucre est un sucre modifié ou un sucre substitué.

4. Composition pour utilisation selon la revendication 3, dans laquelle les molécules amphipathiques comprennent ou consistent en des molécules phospholipidiques, éventuellement dans laquelle les molécules phospholipidiques comprennent des phospholipides chargés négativement, des phospholipides neutres ou une combinaison de ceux-ci, et éventuellement en outre, les molécules phospholipidiques contribuent à une charge nette de 1-3 par molécule d'apolipoprotéine dans l'apomère.

5. Composition pour utilisation selon la revendication 4, dans laquelle les molécules phospholipidiques consistent en une combinaison de phospholipides chargés négativement et neutres, éventuellement dans laquelle le rapport molaire de phospholipide chargé négativement au phospholipide neutre va de 1:1 à 1:3, en outre éventuellement environ 1:1 ou environ 1:2.

6. Composition pour utilisation selon l'une quelconque des revendications 1 à 5, dans laquelle au plus 20 %, au plus 10 %, au plus 5 %, ou au plus 2 % des molécules d'apolipoprotéine dans la composition sont sous forme agrégée et/ou au plus 20 %, au plus 10 %, au plus 5 %, ou au plus 2 % des molécules d'apolipoprotéine dans la composition sont sous forme monomère.

7. Composition pour utilisation selon l'une quelconque des revendications 1 à 6, dans laquelle au moins 75 %, au moins 85 %, au moins 95 %, ou au moins 98 % des particules dans la population présentent un rayon de Stokes inférieur à 3,5 nm.

8. Composition pour utilisation selon l'une quelconque des revendications 1 à 7, dans laquelle :
(a) les apomères dans la population présentent en moyenne 1,8 à 2,5 molécules d'apolipoprotéine et 0,9-2,5 molécules amphipathiques chargées négativement, éventuellement environ 2 molécules d'apolipoprotéine et environ 1 ou environ 2 molécules amphipathiques chargées négativement ;
(b) les apomères dans la population présentent en moyenne 3,5 à 4,5 molécules d'apolipoprotéine et 0,9-2,5 molécules amphipathiques chargées négativement, éventuellement environ 4 molécules d'apolipoprotéine et environ 1 ou environ 2 molécules amphipathiques chargées négativement ; ou
(c) les apomères dans la population présentent en moyenne 7 à 9 molécules d'apolipoprotéine et 0,9-2,5 molécules amphipathiques chargées négativement, éventuellement environ 8 molécules d'apolipoprotéine et environ 1 ou environ 2 molécules amphipathiques chargées négativement.

9. Composition pour utilisation selon l'une quelconque des revendications 1 à 8, dans laquelle :
(a) au plus 20 %, au plus 10 %, au plus 5 %, ou au plus 2 % des molécules amphipathiques sont sous forme non complexée ;
(b) les apomères dans la composition sont homogènes à au moins 75 %, au moins 85 %, au moins 95 %, ou au moins 98 % ; et/ou
(c) des complexes lipoprotéiques présentant des rayons de Stokes supérieurs à 3,4 nm, si présents, représentent au plus 10 %, au plus 5 %, ou au plus 2 % de l'apolipoprotéine dans la composition sur une base pondérale.

10. Composition pour utilisation selon l'une quelconque des revendications 1 à 9, dans laquelle :
(a) les molécules d'apolipoprotéine comprennent ou consistent en des molécules d'apolipoprotéine A-I (ApoA-I), qui sont éventuellement des molécules ApoA-I humaines, qui sont éventuellement recombinantes, éventuellement dans laquelle :
(i) les molécules ApoA-I sont des molécules d'apolipoprotéine A-I_{Milano} (ApoA-I_{M}), d'apolipoprotéine A-I_{Paris} (ApoA-I_{P}), ou d'apolipoprotéine A-I_{Zaragoza} (ApoA-I_{Z}) ; et/ou
(ii) les molécules ApoA-I comprennent une séquence d'acides aminés présentant au moins 90 %, au moins 95 %, au moins 98 %, ou au moins 99 % d'identité de séquence par rapport à SEQ ID NO : 2 ; ou
(b) les molécules d'apolipoprotéine sont des molécules ApoA-II, ApoA-IV, ApoA-V, ApoB, ApoC-I, ApoC-II, ApoC-III, ApoD, ApoE, ApoJ ou ApoH.

11. Composition pour utilisation selon l'une quelconque des revendications 1 à 10, dans laquelle les molécules amphipathiques comprennent un phospholipide chargé négativement qui comprend éventuellement un sel d'un phosphatidylinositol, d'une phosphatidylsérine, d'un phosphatidylglycérol ou d'un acide phosphatidique, éventuellement dans laquelle le sel est un sel de sodium ou un sel de potassium, éventuellement dans laquelle le phospholipide chargé négativement est un sel d'un phosphatidylglycérol, qui est éventuellement un sel de 1,2-dipalmitoyl-sn-glycéro-3-[phospho-rac-(1-glycérol)] (DPPG).

12. Composition pour utilisation selon l'une quelconque des revendications 1 à 11, dans laquelle les apomères comprennent un phospholipide neutre, qui est éventuellement une lécithine ou une sphingomyéline, éventuellement dans laquelle la sphingomyéline est une sphingomyéline d'oeuf, une sphingomyéline végétale, ou une sphingomyéline synthétique, éventuellement dans laquelle les apomères comprennent un phospholipide chargé négativement et un phospholipide neutre, dans laquelle le rapport molaire du phospholipide chargé négativement au phospholipide neutre dans la composition va de 1:1 à 1:3, éventuellement environ 1:1 ou environ 1:2.

13. Composition pour utilisation selon l'une quelconque des revendications 1 à 12, qui est sous forme d'une composition pharmaceutique comprenant un ou plusieurs supports, diluants et/ou excipients pharmaceutiquement acceptables.

14. Composition pour utilisation selon l'une quelconque des revendications 1 à 13, pour utilisation dans le traitement d'un trouble dyslipidémique chez un sujet en ayant besoin, éventuellement dans laquelle le sujet est un être humain, éventuellement dans laquelle :
(a) le sujet présente ou est vulnérable face à l'hyperlipidémie ou une maladie cardiovasculaire, éventuellement dans laquelle ladite hyperlipidémie est une hypercholestérolémie, et éventuellement dans laquelle la maladie cardiovasculaire est une athérosclérose, un accident vasculaire cérébral, un infarctus du myocarde, un syndrome coronarien aigu, une angine de poitrine, une claudication intermittente, une ischémie critique des membres, une sclérose de la valve auriculaire ou une resténose ; ou
(b) le sujet présente un déficit en ApoA-I, un déficit en ABCA1 ou une hypercholestérolémie familiale.

15. Composition pour utilisation selon l'une quelconque des revendications 1 à 13, pour utilisation dans le traitement d'un trouble hépatique chez un sujet en ayant besoin, éventuellement dans laquelle le sujet est un être humain, et éventuellement dans laquelle ledit sujet présente une stéatose hépatique non alcoolique (NAFLD), une stéatohépatite non alcoolique (NASH), ou une hépatite.
